# FASCICULE DE BREVET EUROPEEN

(11) **EP 2 968 660 B1**
(45) Date de publication et mention de la délivrance du brevet: **31.10.2018**
(21) Numéro de dépôt: 14715368.8
(22) Date de dépôt: 14.03.2014
(51) Int. Cl.: A61L 27/12, A61L 27/36, A61L 27/54, A61K 38/18, A61L 24/00, A61L 24/02, A61L 24/10, C07K 14/51

(54) **SUBSTITUTS OSSEUX GREFFES PAR DES PEPTIDES MIMETIQUES DE LA PROTEINE HUMAINE BMP-2**
DURCH MIMETISCHE PEPTIDE DES MENSCHLICHEN BMP-2-PROTEINS GEPFROPFTES KNOCHENERSATZMATERIAL
BONE SUBSTITUTES GRAFTED BY MIMETIC PEPTIDES OF HUMAN BMP-2 PROTEIN

(30) Priorité: 14.03.2013 FR 1352302
(43) Date de publication de la demande: 20.01.2016
(73) Titulaire: Teknimed, 65500 Vic-en-Bigorre (FR)
(72) Inventeur: DURRIEU, Marie-Christine, F-33000 Bordeaux (FR); ZOUANI, Omar, 8840 Einsiedeln (CH)
(74) Mandataire: Cabinet Morelle & Bardou SC
(86) Numéro de dépôt international: PCT/FR2014/050601
(87) Numéro de publication internationale: WO 2014/140504

(56) Documents cités:
- US-A1- 2007 160 681
- US-A1- 2011 129 611

## Description

La présente invention appartient au domaine des matériaux de substitution destinés à la chirurgie osseuse réparatrice des tissus durs, tels que les os, les articulations et les dents.

Elle a pour objet un matériau de substitution osseuse utilisable pour toutes les applications de la chirurgie osseuse, présentant des propriétés ostéoinductrices s'exprimant de manière rapide et intense. Ce matériau est composé d'un support phosphocalcique fonctionnalisé de manière covalente avec une densité contrôlée, par un peptide mimétique de la protéine humaine BMP-2. Un procédé d'obtention de ce matériau est également objet de l'invention.

Les méthodes de traitement conventionnelles pour la réparation des tissus osseux reposent sur la chirurgie et font appel à la pose d'implants, ainsi qu'à l'emploi de ciments de comblement. Ces substituts osseux, qui peuvent être des implants solides préformés, résorbables ou non, ou des matériaux plus ou moins pâteux préparés à partir de granules, de poudres à mélanger avec un solvant et se solidifiant après leur injection dans la zone à traiter, sont communément fabriqués à partir de composés phosphocalciques.

En effet, ces composés sont largement utilisés en chirurgie osseuse car ils apportent une plus grande souplesse mécanique aux implants comparés aux implants métalliques, et il est possible de les formuler comme des pièces solides ou comme des ciments de comblement. Ils sont en outre appréciés pour leur compatibilité cytologique et leur rôle bénéfique dans la formation d'une nouvelle matrice osseuse minérale.

Cependant, la consolidation de l'os peut prendre plusieurs mois. De ce fait, dans de nombreux cas, on observe des blessures, des défauts de régénération tissulaire, ou encore une croissance osseuse insuffisante pour obtenir le résultat souhaité. Pour répondre à ce problème, des recherches ont été menées pour accélérer la régénération de l'os à l'aide de protéines morphogénétiques osseuses (ou BMPs selon l'acronyme anglais pour bone morphogenetic proteins).

Les protéines BMPs sont un groupe de facteurs de croissance connus pour induire la formation des tissus cartilagineux (chondrogenèse) et osseux (ostéogénèse) par différenciation des cellules mésenchymateuses *in vivo.* Elles interagissent avec des récepteurs spécifiques présents à la surface des cellules, ce qui entraîne leur activation.

Parmi les différentes BMPs identifiées à ce jour, la BMP-2 a montré les meilleures capacités ostéogéniques. Cependant, si la protéine BMP-2 a eu un effet prometteur sur animaux, les résultats d'essais cliniques menés sur l'homme sont controversés quant aux effets obtenus (Carragee EJ, Hurwitz EL, Weiner BK. A critical review of recombinant human bone morphogenetic protein-2 trials in spinal surgery: emerging safety concerns and lessons learned. Spine J. 2011, 11, 471 - Kang JD. Another complication associated with rhBMP-2. Spine J. 2011; 11, 517).

Son emploi soulève en outre d'autres problèmes. En particulier, du fait que la protéine BMP-2 a une demi-vie courte et est rapidement métabolisée, l'injection d'une dose relativement élevée est nécessaire pour induire une ostéogenèse suffisante durant un traitement long. Ceci accroît le risque de réactions immunitaires lors de l'administration au patient, qui peut entraîner un niveau de toxicité indésirable. Ce faisant, sa production à grande échelle est longue et très coûteuse. Enfin et surtout, les essais cliniques ont montré des résultats contradictoires quant à l'efficacité d'une administration directe en tant que substance inductrice du processus d'ostéosynthèse.

Deux approches sont actuellement adoptées pour surmonter ces difficultés. La première consiste à transfecter le gène bmp-2 dans des cellules souches mésenchymateuses puis à faire exprimer la protéine BMP-2 par les cellules transgéniques. Cependant, les vecteurs contenant le gène bmp-2 sont pour la plupart des adénovirus qui pourraient être nocifs pour les cellules hôtes. En outre, un risque de mutations existe pour les gènes de l'hôte. L'emploi de vecteurs non viraux élimine ce risque, mais alors l'expression de la protéine BMP-2 est réduite.

La seconde approche consiste à utiliser *in vivo* des peptides synthétiques de longueur inférieure à celle de la protéine complète, imitant l'activité ostéogénique de la protéine BMP-2. Les peptides sont conçus à partir de la séquence connue de la protéine, en recherchant des segments responsables de l'activité ostéogénique, et en modifiant éventuellement la séquence des acides aminés pour moduler, intensifier et renforcer cette activité. Ces peptides peuvent être apportés de différentes manières et sous différentes formes au niveau des zones à traiter.

Le document US 7754683 décrit trois peptides de petite taille (moins de 30 acides aminés), obtenus par adjonction de groupements chimiques à la séquence native de la BMP-2 correspondant au récepteur II (20 acides aminés). Leur activité ostéoinductrice améliorée provient, selon les auteurs, de la présence de groupements anioniques actifs favorisant la minéralisation. Ils peuvent être formulés en solution et injectés directement dans la zone à traiter, ou encapsulés dans des microsphères polymériques à libération progressive, ou encore intégrés dans la chaîne d'un polymère biodégradable pour former un copolymère biodégradable, lequel est introduit dans la zone à consolider. Dans ce mode d'administration, les peptides une fois libérés sont rapidement dispersés et dégradés ce qui implique l'emploi de quantités encore importantes. Une formation osseuse ectopique non contrôlée est à craindre et est malheureusement vérifiée dans de tels systèmes.

Il a aussi été proposé d'utiliser des greffons synthétiques ou des ciments de comblement mimant les propriétés ostéoinductrices se manifestant dans les tissus naturels. Les biomatériaux utilisés en chirurgie osseuse peuvent alors contenir des facteurs de croissance, tels que les protéines morphogénétiques osseuses (BMPs) pour favoriser la régénération osseuse.

Le document US 2007/0160681 par exemple, décrit des matériaux destinés à la greffe osseuse, et des structures pour l'ingénierie tissulaire, qui comportent un peptide de faible poids moléculaire (de 16 à 25 acides aminés), immobilisé à leur surface à raison de 0,1 mg/cm² à 10 mg/cm². Ce peptide peut être un peptide favorisant l'adhésion cellulaire doté d'un motif RGD, ou encore un peptide ayant une activité de facteur de croissance, par exemple un segment d'une protéine telle que la BMP-2, de séquence identique à la séquence de la protéine native. Les auteurs observent des effets remarquables au bout de huit semaines après implantation.

Les résultats obtenus jusqu'à présent montrent que de nombreux paramètres sont en jeu et doivent être pris en compte pour obtenir un système stable bien reconnu par l'organisme et qui stimule efficacement et durablement son activité ostéoinductrice. La connaissance des séquences actives des protéines impliquées n'est pas suffisante.

En effet, la régulation de la différenciation osseuse est multiparamétrique, notamment du fait de l'influence des propriétés mécaniques et biochimiques du microenvironnement.

Plus particulièrement, on sait que la physiologie de la cellule est affectée par une série de composants extracellulaires qui interagissent fortement avec les facteurs de croissance. Or, la coopération entre les substituts osseux et les facteurs de croissance dans ce microenvironnement n'a pas été étudiée jusqu'à présent. Les tentatives faites pour créer des matériaux bioactifs en y greffant des protéines ou des segments peptidiques mimétiques se sont avérées décevantes.

Parmi les raisons pouvant être avancées à ce sujet, on peut retenir que les protéines macromoléculaires qui adhèrent à la surface d'un matériau ont tendance à prendre différentes structures tertiaires non prévisibles à partir de leur conformation initiale à l'état libre, ce qui peut entraîner une activité biologique réduite et inhomogène en raison d'une exposition insuffisante des sites actifs. Ceci est également valable pour les peptides à l'état soluble. Lors de l'immobilisation des peptides par un greffage chimique covalent, le risque est grand qu'un mauvais repliement se produise, rendant les sites actifs inaccessibles.

Il existe donc un besoin non satisfait à ce jour, de disposer d'un matériau utilisable en ostéochirurgie remplissant le rôle de substitut osseux et qui permette en même temps de promouvoir l'ostéoinduction et d'accélérer la formation osseuse, indépendamment de la condition physique particulière de chaque patient, sans risque immunitaire ou toxique.

Les auteurs de la présente invention ont considéré que, pour une séquence particulière du peptide, la nature du support va jouer un rôle facilitant, ou au contraire répresseur, de l'activité ostéoinductrice. L'état des surfaces du support, la nature et la quantité des sites réactifs en surface, la densité du greffage, la nature de la liaison entre le peptide et le support, sont certainement des paramètres à prendre en compte dans la conception du substitut osseux répondant aux exigences précédemment énoncées. Les modes de préparation de substituts osseux associés à des facteurs de croissance influent forcément sur ces phénomènes.

C'est dans ce contexte que les inventeurs ont mis au point un modèle expérimental pour caractériser le microenvironnement responsable de la différenciation cellulaire du lignage ostéoblastique. Dans le cadre de ces travaux, il a été trouvé qu'un segment peptidique déterminé peut induire une activité ostéoinductrice élevée, lorsqu'il est greffé de manière covalente sur un matériau phosphocalcique, avec une densité particulière. De manière surprenante, ce segment peptidique de taille réduite (une vingtaine d'acides aminés seulement) présente une efficacité élevée malgré un environnement fort différent de son environnement naturel. C'est un mime peptidique qui interagit avec son récepteur par des liaisons de types ionique, hydrogène et hydrophobe. De manière encore plus surprenante, il a été mis en évidence que l'activité maximale ne se manifeste pas quand la densité peptidique augmente, mais pour des densités de peptides greffés en surface du matériau correspondant à des valeurs de l'ordre de quelques picomoles ou quelques dizaines de picomoles par mm².

Un but de la présente invention est ainsi de proposer un matériau implantable en tant que substitut osseux, ayant une action ostéoinductrice accrue, plus intense et plus rapide. Un autre but de l'invention est d'obtenir avec ce matériau un effet réparti de manière homogène dans toute la zone de cicatrisation, et perdurant suffisamment longtemps pour assurer une consolidation complète. Encore un but de l'invention est de disposer d'un substitut osseux pouvant être mis en oeuvre aisément, tant pour la fabrication de dispositifs prothétiques et d'implants divers, que pour la préparation de ciments de comblement. Un autre but de l'invention est de disposer d'un matériau de substitution osseuse greffé par un peptide mimétique de la protéine BMP-2, avec une concentration minimale et optimale. Bien entendu, ce matériau doit respecter toutes les normes de sécurité sanitaire et de (non) toxicité. Il doit aussi pouvoir être produit dans des conditions économiquement acceptables.

Les objectifs ci-dessus sont atteints par la présente invention, laquelle a pour objet un matériau de substitution osseuse pour la chirurgie osseuse et dentaire, lequel comprend :
i) un support solide à base d'au moins un composé phosphocalcique présentant des groupements hydroxyles libres en surface, et
ii) une quantité d'un peptide mimétique de la protéine humaine BMP-2 de séquence
   KIPKACCVPTELSAISMLYL
   (SEQ ID No 1), ou d'un dérivé de ce peptide qui est un ligand interagissant avec le récepteur cellulaire BMP de type II, ledit dérivé ayant une séquence
   KX₁PKX₂Z₁Z₂X₃PTEX₄SAISMLYL
   (SEQ ID No 3) dans laquelle X_{1;} X₂, X₃ et X₄ sont des acides aminés non polaires, identiques ou différents et Z₁ et Z₂ identiques ou différents, désignent un résidu cystéine ou sérine,
   ladite quantité de peptide mimétique de la protéine BMP-2 étant greffée de manière covalente par son extrémité N-terminale au niveau desdits groupements hydroxyles, avec une densité inférieure à 100.10⁻¹² mol/mm² de surface du support solide.

Les supports solides pouvant être utilisés pour la présente invention sont des composés phosphocalciques. Ces composés présentent naturellement des fonctions hydroxyles en surface, en nombre important mais variable selon leur nature exacte. Ceux qui sont utilisés en chirurgie osseuse sont essentiellement des hydroxyapatites et du phosphate tricalcique possédant tous des fonctions OH disponibles, mais d'autres composés pourraient aussi convenir.

Le peptide est un peptide mimétique de la protéine humaine BMP-2 native. La protéine humaine BMP-2 est bien connue pour son rôle dans la différentiation osseuse, en tant que ligand du récepteur cellulaire de type II. Sa séquence complète a été établie depuis plusieurs années (Zouani OF, Chollet C, Guillotin B, Durrieu MC. Differentiation of pre-osteoblast cells on poly(ethylene terephthalate) grafted with RGD and/or BMPs mimetic peptides. Biomaterials. 2010 Nov;31(32):8245-53). La séquence peptidique SEQ ID No1 est un segment de la protéine native, qui comprend 20 acides aminés. Le peptide selon l'invention peut être un segment de 20 acides aminés mimétique de la protéine BMP-2, spécialement sélectionné pour son activité ostéoinductrice. Il est qualifié de mimétique car il constitue un ligand interagissant avec le récepteur cellulaire BMP de type II. Il peut être identique ou être un homologue dérivé du peptide natif à savoir de même taille que celui-ci mais dans lequel certains acides aminés ont été remplacés.

Ainsi, le peptide mimétique selon l'invention peut être le peptide défini par la séquence SEQ ID No 1 ou tout peptide dérivé défini par la séquence SEQ ID No 3, intégrant une possible substitution d'au plus quatre acides aminés, notés X_{1;} X₂, X₃ et X₄, qui sont des acides aminés non polaires, identiques ou différents entre eux. Les acides aminés non polaires sont notamment les acides aminés naturels choisis parmi la glycine, l'alanine, la valine, la leucine, la méthionine ou l'isoleucine. On peut par exemple choisir une combinaison où X₁ = Ile, X₂ = Ala, X₃ = Val et X₄ = Leu ; ou encore X₁ = Leu, X₂ = Val, X₃ = Leu et X₄ = Ala.

Les dérivés selon la présente invention sont tels qu'ils conservent la propriété de ligand du récepteur cellulaire BMP de type II que possède le peptide de séquence SEQ ID No 1. Le procédé de fabrication préféré du peptide mimétique est la synthèse chimique. Toute technique connue de l'homme de l'art peut être utilisée. Par exemple, le peptide peut être synthétisé en phase solide par la méthode de Krieger DE et al., Proc Nat Acad Sci U.S.A. 1976; (73(9):3160-4.

Le segment peptidique est greffé de manière covalente, ce qui signifie qu'il est immobilisé sur la matrice minérale du support solide. Ce peptide comprend en particulier la séquence peptidique de six acides aminés ISMLYL, qui est apparue aux inventeurs comme décisive pour que l'activité ostéoinductrice se manifeste. De ce fait, il est avantageusement fixé par son extrémité N-terminale au niveau desdits groupements hydroxyles du support, généralement par l'intermédiaire d'un agent de couplage qui sera décrit plus loin.

De manière surprenante, il a été trouvé que pour obtenir un effet satisfaisant, la densité de peptides greffés au support doit être inférieure à 100.10⁻¹² mol/mm² de surface (soit, pour le peptide selon la première combinaison susmentionnée dont la masse molaire est de 2237 g/mol, une densité inférieure à 22,35.10⁻³ mg/cm² de surface). Le système s'est révélé avoir une activité ostéoinductrice marquée très rapidement (dans les premières heures) après implantation. Les peptides greffés sont stables et capables d'induire durablement une fonction d'induction ostéogénique. On peut penser que cette action très rapide et intense est liée à une densité de peptides optimale mais aussi à la préservation d'une conformation tridimensionnelle correcte. Une densité plus forte pourrait affecter le repliement du peptide et donc son activité. Il est précisé que la densité de peptide greffé, n'est pas nulle. On pourra cependant greffer des quantités très faibles pouvant aller jusqu'à 0,2 pmol/mm² de surface, tout en conservant une activité inductrice des récepteurs BMP.

Cependant, si une densité très faible est à même d'activer les récepteurs BMP de la membrane de la cellule, elle peut ne pas être suffisante pour induire une différenciation accélérée et intense. C'est pourquoi, selon une caractéristique avantageuse de l'invention, la densité prédéterminée dudit peptide greffé à la surface du support solide est inférieure à 10.10⁻¹² mol/mm².

De manière préférée, elle est comprise dans l'intervalle allant de 4,5.10⁻¹² mol/mm² à 7,5.10⁻¹² mol/mm². De manière encore préférée, cette densité molaire peut être de l'ordre de 5,9.10¹² mol/mm², ce qui correspond pour le peptide de séquence SEQ ID No 2, à une densité massique de l'ordre de 1,32.10⁻³ mg/cm².

On note par ailleurs que la nature exacte ou la structure du matériau support pouvant jouer un rôle significatif sur l'activité peptidique, il se peut que pour différents matériaux, les densités optimales de greffage soient différentes : grâce à la présente invention, l'homme du métier est en mesure de contrôler le greffage du peptide à une densité prédéterminée biologiquement optimale. Cette possibilité découle du mode de greffage en deux étapes qui sera décrit en détail plus loin, et qui est également objet de la présente invention.

Selon une caractéristique avantageuse du matériau selon l'invention, une cystéine est fixée à l'extrémité N-terminale du peptide. De la sorte, une fonction thiol est disponible à l'extrémité du peptide mimétique, qui pourra aisément réagir avec un site présent sur le support solide, induisant par la même occasion l'orientation correcte du peptide durant le greffage. Afin que cette cystéine terminale ait l'exclusivité de la réaction de greffage, il est préférable que d'autres acides aminés présents dans le peptide ne réagissent pas avec le site de fixation du support solide.

C'est pourquoi, selon une autre caractéristique avantageuse du matériau selon l'invention, dans ledit peptide mimétique, Z₁ et Z₂ sont deux résidus sérine. Cette substitution s'est avérée d'autant plus opportune qu'elle a permis de remédier à la formation spontanée de couches peptidiques en surface du support. Ce phénomène, attribué à la formation de ponts disulfures entre les cystéines de différents peptides, a été identifié comme une limitation importante à l'accessibilité du peptide, et a ainsi été surmonté par la présente invention.

Ainsi, dans une variante préférée, le peptide contenant le peptide mimétique selon l'invention, augmenté d'une cystéine de liaison (formant un "linker"), a une séquence de 21 acides aminés, conforme à SEQ ID No 2 :
Nterminus-CKX₁PKX₂SSX₃PTEX₄SAISMLYL-Cterminus

Ce peptide est linéaire et comprend la séquence de six acides aminés ISMLYL.

Selon un mode de réalisation avantageux de l'invention, le peptide est lié au support solide par l'intermédiaire d'un agent de couplage bifonctionnel comprenant un agent de fonctionnalisation de surface associé à un agent de liaison. Ledit agent de couplage bifonctionnel peut comprendre un organosilane, de préférence le (3-aminopropyl)-triéthoxysilane (noté APTES), lequel peut être associé au N-succinimidyl-3-maléimidopropionate (encore appelé ou réticulent SMP). De manière connue, un espaceur peut être introduit. Finalement, selon ce mode de réalisation, le peptide mimétique est lié au support solide par l'intermédiaire d'une cystéine fixée à son extrémité N-terminale et d'un agent de couplage bifonctionnel comprenant un agent de fonctionnalisation de surface associé à un agent de liaison.

Les supports solides mis en oeuvre pour réaliser le matériau objet de la présente invention sont à base de composés phosphocalciques, qui sont aptes à être utilisés dans le domaine de la chirurgie osseuse, y compris en dentisterie. Leur structure chimique est telle qu'ils possèdent des groupements hydroxyles libres en surface, bien que dans des proportions variables. Les supports solides peuvent être constitués d'un seul composé, par exemple une hydroxyapatite, ou avoir une composition biphasique, comme c'est le cas des mélanges d'hydroxyapatite et de phosphate de calcium.

C'est pourquoi, selon un mode particulier de réalisation du matériau objet de la présente invention, ledit au moins un composé phosphocalcique est de l'hydroxyapatite (HAP), du phosphate tricalcique (TCP), ou un mélange de ceux-ci. D'autres matériaux phosphocalciques connus de l'homme du métier peuvent également être utilisés. On note que des additifs ou autres ingrédients peuvent être ajoutés au composé phosphocalcique, soit pour les besoins du procédé de fabrication, soit à des fins thérapeutiques (vitamines, antibiotiques par exemple). L'ajout de ces additifs, qui sont toujours employés en quantités minimes, est connu et ne fera pas l'objet d'une description particulière dans le cadre de la présente invention.

De manière particulièrement intéressante, il a été trouvé que le matériau greffé par le peptide mimétique objet de l'invention peut être obtenu à partir d'un support solide, quel que soit son degré de fractionnement et sa forme cristalline. Il peut notamment être obtenu à partir d'un support cristallin poreux, tel qu'une céramique, mais aussi à partir d'un support amorphe tel qu'une poudre d'un composé phosphocalcique, ou à partir de nanoparticules phosphocalciques.

Selon un mode de réalisation de l'invention, le support solide est une céramique sous forme de granulés ou de blocs. Dans ce cas, la taille des objets soumis au greffage peut être de type granulés (c'est-à-dire de l'ordre de un à quelques millimètres) ou de la taille d'un bloc (c'est-à-dire de l'ordre de quelques millimètres ou du centimètre). Cette caractéristique est particulièrement avantageuse pour préparer des matériaux selon l'invention sous la forme de blocs massifs préformés, pouvant remplacer un greffon à implanter pour combler une lacune importante de la structure osseuse. Elle ouvre également la possibilité d'une application en ingénierie tissulaire, où le matériau pourra être utilisé comme matériau support pour une croissance tissulaire avant implantation (appelé scaffold en anglais), afin de favoriser la régénération osseuse.

Il convient de souligner que, de manière inattendue et très avantageuse, le matériau obtenu sous forme de bloc massif présente une activité ostéoinductrice tout aussi élevée que celle des formes plus fractionnées (poudre ou granulés), indiquant que le peptide a été greffé dans la masse. Il se pourrait donc que pour un composé céramique ayant une porosité comprise entre 55% et 75% en volume avec des pores de 300 à 500 microns, la surface de la porosité reste accessible en profondeur au peptide, et ce malgré la sensibilité de ce type de molécule aux diverses interactions physiques et chimiques fortes qui étaient attendues dans tel un environnement.

Selon un autre mode de réalisation de l'invention, le support solide est sous la forme d'une poudre amorphe ou de nanoparticules. Dans ce cas, la taille des particules constituant le support solide peut être de quelques micromètres pour les poudres, et peut aller de quelques dizaines de nanomètres jusqu'à environ 200 nm pour les nanoparticules. La surface présentant des groupements hydroxyles disponibles pour réagir avec le peptide d'intérêt est donc très élevée.

Avantageusement, le matériau selon l'invention peut se présenter avec un support solide sous la forme d'une poudre amorphe ou de nanoparticules en suspension aqueuse. Des ciments et des nanogels (gels de nanoparticules en suspension) de comblement osseux peuvent ainsi être formulés. Ces matériaux pourront être stockés sous cette forme et mis en oeuvre directement par le praticien.

Finalement, on pourra utiliser pour la préparation du matériau innovant, un support solide déjà connu et utilisé par les chirurgiens, qui n'auront ainsi pas à modifier leurs protocoles opératoires. De la sorte, les chirurgiens disposent d'une gamme de matériaux leur permettant de choisir la forme la plus adéquate pour l'acte chirurgical envisagé, aucune adaptation particulière n'étant nécessaire par rapport aux produits auxquels ils ont l'habitude de recourir. Ceci représente un avantage non négligeable de l'invention. Quel que soit le support solide choisi, il offre en surface une grande quantité de sites OH pouvant être greffés.

Le matériau décrit ci-dessus, comprenant un substitut osseux présentant des fonctions hydroxyles libres, associé avec son peptide mimétique de la BMP-2 immobilisé de manière covalente par son extrémité N-terminale, induit une rapide différenciation et une prolifération ostéoblastique. Par ailleurs, il induit une forte interaction entre les cellules de la moelle osseuse telles que les cellules souches mésenchymateuses, les ostéoblastes et les cellules vasculaires. Des clusters cellulaires sont formés qui témoignent d'un pouvoir régénérateur intense de ces matériaux greffés.

Les résultats obtenus témoignent donc de la création d'un microenvironnement cellulaire comprenant les peptides mimétiques de la BMP-2, qui favorise la régénération osseuse dans une courte période de temps. Elle trouve son application dans tous les domaines de la chirurgie osseuse (musculo-squelettique et dentaire), notamment en orthopédie, en neurochirurgie, en chirurgie maxillo-faciale, en stomatologie, en oncologie, implantologie, vertébroplastie, ou autre ... Le matériau peut en outre être utilisé en tant que tel, ou bien combiné à d'autres types de supports et de biomatériaux, par exemple à des matériaux composites avec matrice polymère.

La présente invention vise également un protocole de préparation d'un matériau constituant un tel microenvironnement cellulaire. Le matériau selon l'invention peut être préparé à partir de différents substrats phosphocalciques, céramiques, poudres amorphes ou nano-cristaux, par greffage d'un peptide tel que défini précédemment, à condition que le procédé mis en oeuvre permette de contrôler la quantité de peptide mimétique de la protéine BMP-2 qui est greffée de manière covalente par son extrémité N-terminale au niveau desdits groupements hydroxyles, de sorte qu'elle soit inférieure à 100 pmol/mm² de surface du composé phosphocalcique. Pour ce faire, un procédé a été mis au point qui est également un objet de la présente invention.

Ainsi, selon l'invention, est revendiqué un procédé de préparation d'un matériau de substitution osseuse selon l'une des revendications précédentes, comprenant i) un support solide à base d'au moins un composé phosphocalcique présentant des groupements hydroxyles libres en surface, et ii) une quantité d'un peptide mimétique de la protéine humaine BMP-2 de séquence
KIPKACCVPTELSAISMLYL
(SEQ ID No1), ou d'un dérivé de ce peptide qui est un ligand du récepteur cellulaire de type II, ledit dérivé ayant une séquence
KX₁PKX₂Z₁Z₂X₃PTEX₄SAISMLYL
(SEQ ID No 3) dans laquelle X₁, X₂, X₃ et X₄ sont des acides aminés non polaires, identiques ou différents et Z₁ et Z₂ identiques ou différents, désignent un résidu cystéine ou sérine,
le procédé comprenant essentiellement les étapes consistant à :
a) faire réagir une partie des groupements hydroxyles dudit composé phosphocalcique avec un agent de fonctionnalisation puis avec un agent de liaison pour obtenir un support solide ayant une densité prédéterminée de sites modifiés en surface,
b) faire réagir le support solide ainsi modifié avec une solution contenant ledit peptide en excès pour obtenir un matériau ayant ladite densité prédéterminée inférieure à 100.10⁻¹² mol/mm² (soit 100 pmol/mm²) de sites greffés par ledit peptide.

L'étape a), qui comporte deux réactions successives, permet de réaliser une modification d'un nombre déterminé de sites hydroxyles libres présents à la surface du composé phosphocalcique, à l'aide d'un agent de couplage bifonctionnel réalisé par un agent de fonctionnalisation auquel on associe un agent de liaison qui sera alors apte à réagir avec le peptide d'intérêt. L'étape b) réalise la réaction entre l'agent de liaison et le peptide mimétique. La densité de groupes hydroxyles modifiés à l'étape a) est contrôlée par la maîtrise des conditions réactionnelles, en particulier les durées d'incubation successives du support avec les deux agents réactifs. Les différents composés phosphocalciques ayant naturellement plus ou moins de sites OH libres, le temps de réaction sera ajusté pour que soit modifiée une quantité de sites voulue définie au préalable. Dans un second temps, le peptide d'intérêt est greffé, dans des conditions réactionnelles telles que les sites précédemment modifiés vont réagir avec le peptide. Même si tous les sites modifiés du support ne réagissent pas avec le peptide mimétique, il n'en reste pas moins qu'une certaine proportion définie de sites modifiés est greffée avec le peptide, cette proportion étant reproductible dans des conditions opératoires fixées. C'est donc à l'étape a) qu'est déterminée la densité de greffage qui sera finalement obtenue. La densité de sites modifiés à l'issue de l'étape a) est généralement supérieure à la densité de sites greffés par le peptide à l'issue de l'étape b).

Les conditions du procédé sont donc ajustées pour obtenir un support solide ayant une densité prédéterminée de sites modifiés puis greffés en surface. Dans ces conditions, il semble que la formation d'une mono-couche de peptides est favorisée, avec un encombrement stérique maîtrisé et sans interférence marquée entre groupes peptidiques voisins, de sorte que le repliement des peptides n'est pas affecté.

Selon une caractéristique préférée du procédé objet de l'invention, à l'étape a) on fait réagir une partie des groupements hydroxyles dudit composé phosphocalcique avec un agent de fonctionnalisation puis avec un agent de liaison pour obtenir, à l'étape b) un support solide ayant une densité prédéterminée de sites greffés en surface inférieure à 10.10⁻¹² mol/mm². De préférence, la densité prédéterminée de sites greffés en surface va de 4,5.10⁻¹² mol/mm² à 7,5.10⁻¹² mol/mm²

On crée ainsi un support modifié avec un assemblage constituant un agent de couplage bifonctionnel. Il est formé par exemple d'un composé choisi parmi les organosilanes, tel que le (3-aminopropyl)-triéthoxysilane (ou APTES), qui peut être associé à un agent de liaison de la famille des succinimidyls, tel que le N-succinimidyl-3-maléimidopropionate (réticulent SMP) ou un dendron contenant plusieurs fonctions maléimides.

Dans un mode particulier de mise en oeuvre du procédé objet de l'invention, à l'étape a) on place le support solide dans une solution de (3-aminopropyl)-triéthoxysilane, puis dans une solution de N-succinimidyl-3-maléimidopropionate, pour obtenir ladite densité prédéterminée de sites hydroxyles modifiés en surface dudit support solide. Par exemple, on peut utiliser une solution de SMP à 2.10⁻³ M dans le DMF.

Selon une autre caractéristique possible du procédé objet de l'invention, à l'étape b) on place le support solide modifié dans une solution aqueuse contenant ledit peptide à une molarité comprise entre 10⁻⁵M et 10⁻²M, pendant 15 à 30 heures. De manière avantageuse, on peut travailler avec une solution aqueuse de peptide à 10⁻³M, qui est alors en large excès. La durée d'incubation est suffisante pour que la réaction des peptides avec les sites modifiés à l'étape a) soit maximale.

De préférence selon l'invention, ledit peptide est greffé de manière covalente sur les sites modifiés à l'étape a) par son extrémité N-terminale. Si le peptide a une cystéine en position N-terminale, c'est le groupe thiol de la cystéine qui réagit sur la partie maléimide du réticulent SMP.

Selon un mode de réalisation préféré de l'invention, avant chacune des deux réactions de l'étapes a) le support solide est séché, à une température de l'ordre de 70 °C à 100°C. Le séchage est effectué dans un four thermostaté et se prolonge quelques heures. On se débarrasse ainsi de toute trace de solvant et notamment de l'eau potentiellement présente autour, et le cas échéant dans la porosité de support solide, ce qui améliore le rendement des réactions avec une reproductibilité élevée.

En effet, à l'étape a), la réaction de l'agent de fonctionnalisation avec le matériau support en présence de traces d'eau conduit à la formation d'un réseau de polysiloxanes qui perturbe la fixation ultérieure des peptides. On peut éviter l'apparition de ce réseau tridimensionnel de chaînes polymériques, en chauffant le matériau. Dans certaines variantes du procédé, on peut opérer sous vide secondaire défini comme correspondant à des pressions de 10⁻³ à 10⁻⁷ mbar (soit de 0,1 à 10⁻⁵ pascal). Ces modalités jouent donc un rôle notable pour obtenir une monocouche.

Selon une caractéristique avantageuse du procédé innovant, on réalise après l'étape b), un rinçage à l'eau du matériau de substitution osseuse obtenu, jusqu'à élimination complète des peptides en excès, ce qui inclut ici les peptides non greffés mais juste adsorbés. De la sorte, on élimine les peptides n'ayant pas formé de liaison covalente avec l'agent de liaison. Cette phase de rinçage peut avoir une importance sensible dans l'activité ostéoinductrice que va manifester le matériau. On a émis l'hypothèse à ce sujet que les sites greffés pouvaient être masqués par des composés adsorbés mais non immobilisés de façon covalente, notamment des peptides en excès, que l'organisme du patient devrait éliminer avant que les segments peptidiques greffés soient accessibles et puissent entrer en action.

Le procédé de greffage peut aussi être réalisé en milieu aqueux, selon la même démarche en deux grandes étapes. Dans ce cas, on procède comme suit, à l'étape a) : ajout de l'agent de fonctionnalisation (APTES) en milieu acide, puis ajout d'un agent de liaison (SMP), rinçage et séchage ; puis à l'étape b) : ajout des solutions peptidiques BMP-2, et rinçages pendant 1 semaine.

Selon une caractéristique préférée du procédé de préparation du matériau de substitution osseuse selon l'invention, le composé phosphocalcique est de l'hydroxyapatite (HAP), du phosphate tricalcique (TCP), ou un mélange de ceux-ci. L'homme du métier sait fabriquer de tels composés et leurs mélanges. Il est également connu de choisir l'un ou l'autre en fonction de l'usage visé. Ainsi, ledit support solide peut être choisi parmi :
- une céramique formulée en granulés ou en blocs, pour obtenir ledit matériau de substitution osseuse sous la même forme,
- une poudre amorphe apte à être mise en suspension dans une phase aqueuse pour obtenir ledit matériau de substitution osseuse sous forme d'un ciment de comblement,
- des nanoparticules aptes à être mises en suspension dans une phase aqueuse pour obtenir ledit matériau de substitution osseuse sous forme d'un gel de comblement.

La présente invention sera mieux comprise, et des détails en relevant apparaîtront, grâce à la description qui va être faite de certains modes de réalisation, en relation avec les figures annexées, dans lesquelles :
La Fig. 1 présente le spectre atomique de l'azote d'un matériau greffé par un peptide, objet de l'invention (Fig. 1B) et du support solide avant greffage (Fig. 1A). La Fig. 1C est un cliché en microscopie à fluorescence illustrant la présence du peptide greffé sur ce même matériau.
La Fig. 2 présente des images de microscopie confocale illustrant la différenciation de cellules souches mésenchymateuses humaines de moelle osseuse après 48h au contact d'un matériau selon l'invention.
La Fig. 3 est un graphique illustrant le niveau d'expression du facteur Runx2 et du facteur MyoD après 62h de contact des mêmes cellules avec deux matériaux selon l'invention.
La Fig. 4 présente des images de microscopie confocale illustrant la différenciation de cellules pré-ostéoblastiques après 48h au contact de deux matériaux selon l'invention.
La Fig. 5 est une représentation quantitative de l'activité de la phosphatase alcaline après 48 heures de culture des mêmes cellules avec les mêmes deux matériaux.
La Fig. 6 est un graphique illustrant le niveau d'expression du facteur Runx2 après 48h de contact des mêmes cellules avec les mêmes deux matériaux selon l'invention.
La Fig. 7 représente des images en microscopie confocale de cellules endothéliales cultivées durant 48 heures au contact de deux matériaux selon l'invention.
La Fig. 8 est un graphique montrant la prolifération des cellules endothéliales après 48 heures de culture cellulaire au contact des mêmes deux matériaux.
La Fig. 9 est un graphique montrant l'activité de la phosphatase alcaline de tri-cultures cellulaires après 62 heures au contact de deux matériaux selon l'invention.
La Fig. 10 est un graphique montrant la prolifération des précurseurs ostéoblastiques après 62 heures de culture cellulaire sur ces mêmes matériaux.
La Fig. 11 est un graphique illustrant le niveau d'expression du facteur Runx2 après 62h de contact des mêmes cellules avec les deux mêmes matériaux.
La Fig. 12 présente le spectre atomique de l'azote d'un matériau nanoparticulaire greffé par un peptide objet de l'invention (Fig.12B) et du support solide avant greffage (Fig. 12A). La Fig. 13 représente le taux d'expression du facteur Runx2 par RT-PCR en fonction de la densité du peptide P1 greffé en surface d'un support modèle PET.

### EXEMPLE 1 : Matériau céramique greffé par un peptide mimétique P1

### Support :

Dans le mode de réalisation présenté ci-après, le support solide est un matériau céramique contenant 65% d'hydroxyapatite et 35% de TCP. Le matériau a une porosité de l'ordre de 65% à 75%. Il est disponible dans le commerce, par exemple sous la marque CERAFORM® de TEKNIMED. Il est utilisé sous forme de cubes de 6 mm de côté.

### Peptide :

Le peptide P1 de séquence : CKIPKASSVPTELSAISMLYL

C'est un peptide linéaire découlant de la SEQ ID No 3 :
KX₁PKX₂Z₁Z₂X₃PTEX₄SAISMLYL, augmenté d'une cystéine, avec X₁ = Ile, X₂ = Ala, X₃ = Val et X₄ = Leu, et Z₁ = Z₂ =Ser.

### Mode opératoire (Etape a)

(1) Mettre le support sous vide (10⁻⁵ torr) à 100°C pendant 16 heures.
(2) Sous atmosphère Argon, mettre le support avec l'APTES à 10⁻²M dans l'hexane anhydre pendant 2 heures.
(3) Rincer avec sonication.
(4) Sécher sous vide (10⁻⁵ torr) à 70 °C pendant 16h.
(5) Ajouter l'agent de liaison SMP à une concentration de 2.10⁻³M dans le DMF et incuber pendant 2 heures sous atmosphère Argon.
(6) Rincer plusieurs fois dans le DMF, puis sécher à 70 °C sous vide (10⁻⁵ torr).

On obtient un support modifié.

### Mode opératoire (Etape b)

(7) Ajouter la solution peptidique du peptide P1 (10⁻³M) au support modifié ainsi obtenu et incuber sous atmosphère normale à température ambiante pendant 18h.
(8) Effectuer des rinçages pendant une semaine.

On obtient le matériau M1 greffé par P1 (cube de 6 mm de côté).

### Caractérisation : présence de la séquence peptidiaue

La composition atomique de la céramique greffée M1 a été comparée à celle de la céramique non traitée (support solide pris comme référence). Les mesures ont été réalisées par spectroscopie à photoélectrons aux rayons X (Tableau 1).

**Tableau 1**

| **atome** | **Référence (% atomique)** | **Matériau M1 (% atomique)** |
|---|---|---|
| P2p | 10,5 | 10,55 |
| C1s | 33,5 | 34,52 |
| Ca2p | 15,59 | 13,93 |
| O1s | 39,4 | 38,39 |
| N1s | non détecté | **2,61** |

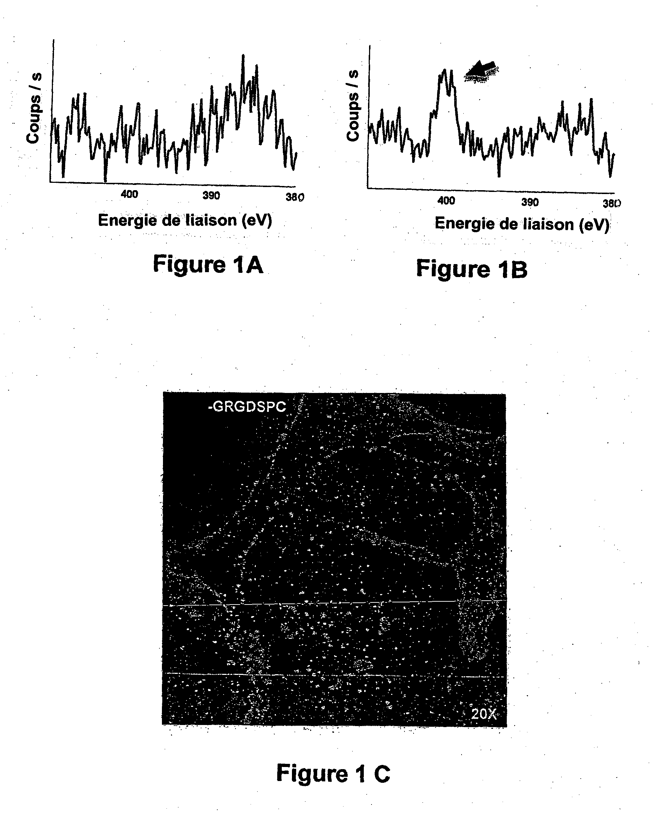

Le spectre d'azote (N1s) en surface des deux matériaux (M1 et référence) obtenu par spectroscopie à photoélectrons aux rayons X est présenté aux Fig. 1A et 1B.

Les résultats montrent l'apparition dans le matériau M1, d'un spectre d'azote à environ 3%, prouvant la présence de séquence peptidique sur le support céramique.

### Dans la masse :

Des cubes du matériau M1 obtenu comme décrit ci-dessus ont été fractionnés en un granulat de moins de 1 mm de diamètre, et soumis à un rinçage pendant 1 mois. Différents points relevés par spectroscopie à photoélectrons aux rayons X ont montré la présence d'azote à hauteur d'environ 1,5%, alors qu'aucune trace d'azote n'est détectée dans les supports céramiques non modifiés.

On a donc une greffage en profondeur du support céramique M1.

### Caractérisation : densité de peptide greffé

Le matériau céramique M1 a été caractérisé afin de déterminer la densité exacte de peptides greffés en surface du support solide. Pour cela, un peptide fluorescent P1f a été préparé et greffé au support solide pour obtenir un matériau M1f. Le peptide P1f est le peptide P1 augmenté à son extrémité C terminale d'un fluorochrome, ici l'isothiocyanate de fluorescéine (en abrégé FITC).

La densité moyenne de peptide P1f en surface du matériau M1 mesurée par microscopie à fluorescence est de 5,9 pmol/mm².

### EXEMPLE 2 : Matériau céramique greffé par un peptide mimétique P2

### Support :

Le support solide est un cube de céramique (65% d'hydroxyapatite et 35% de TCP), identique à celui de l'Exemple 1.

### Peptide :

Le peptide P2 de séquence : CKLPKVSSLPTEASAISMLYL.

C'est un peptide conforme au peptide de séquence :
KX₁PKX₂Z₁Z₂X₃PTEX₄SAISMLYL (SEQ ID No 3), augmenté d'une cystéine,
avec X₁ = Leu, X₂ = Val, X₃ = Leu et X₄ = Ala, et Z₁ = Z₂ =Ser.

### Mode opératoire :

Le protocole de greffage est identique à celui de l'Exemple 1.

On obtient le matériau greffé M2.

### Caractérisation de M2 :

Les résultats sont similaires à ceux obtenus pour le matériau M1.

### EXEMPLE 3 : Activité ostéogénique des matériaux céramiques greffés M1 et M2

L'activité des matériaux M1 et M2 décrits et préparés conformément aux exemples ci-dessus a été évaluée au contact de trois types cellulaires séparément et ensemble au sein d'une même culture cellulaire. Les trois types cellulaires sont les cellules présentes majoritairement au sein de la moelle osseuse. Il s'agit des cellules souches mésenchymateuses, de cellules pré-ostéoblastiques et de cellules endothéliales.

### 1- Activité vis-à-vis des cellules souches mésenchymateuses

Des cellules souches mésenchymateuses humaines de la moelle osseuse (fournies par la société LONZA) ont été cultivées pendant 96 heures sur des supports céramiques non modifiés et des supports céramiques M1 et M2 greffés respectivement par les peptides P1 et P2.

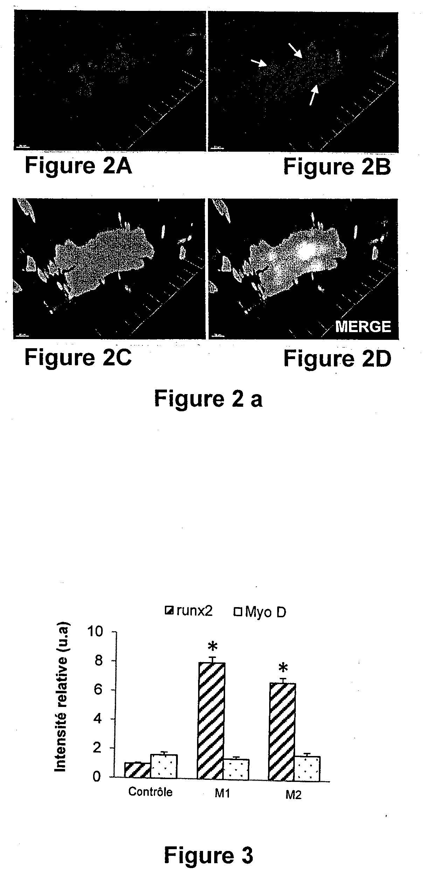

L'engagement ostéogénique des cellules après 48 heures de culture sur les matériaux M1 et M2 greffés par le peptide P1 et P2 a été repéré par microscopie confocale. Les images obtenues révèlent la forme cellulaire grâce à un marquage cytoplasmique par le CMFDA (Fig. 2C), le facteur de transcription ostéogénique maître Runx2 étant marqué par un anticorps anti-Runx2, représenté à la Fig. 2B, et le noyau étant marqué par le DAPI (Fig. 2A), et un cliché fusionné (Merge, Fig 2D). On constate que l'ensemble des cellules souches mésenchymateuses exprime de manière très intense le facteur de transcription Runx2 témoignant d'un engagement des cellules vers un lignage ostéoblastique.

Une quantification du taux d'expression de ce facteur Runx2 dans les matériaux M1 et M2 par rapport à un support céramique non modifié a été établie par quantification de la fluorescence à partir d'images confocales sur au moins 60 cellules. Différentes régions du matériau ont été inspectées au centre, et en périphérie), un effet homogène de différenciation cellulaire a été observé. Le facteur de transcription myogénique MyoD, témoignant d'un engagement cellulaire myoblastique a été effectué lors du marquage cellulaire en tant que contrôle négatif.

Les mesures ont été faites 62 heures après la mise en contact des cellules et des matériaux M1 et M2. Les résultats obtenus sont rapportés sur la Fig. 3. On trouve 80% d'augmentation de l'expression du facteur Runx2 pour le matériau M1 et 68% pour le matériau M2, par rapport au matériau de référence. Aucune trace d'augmentation de l'expression du facteur de transcription MyoD n'a été observée. (**P*< 0.01).

### 2- Activité vis-à-vis des cellules pré-ostéoblastiques

Des cellules pré-ostéoblastiques (la lignée MC3T3-E1, commercialisée par ATCC) ont été cultivées sur les matériaux M1 et M2. Le support solide non greffé est pris comme référence.

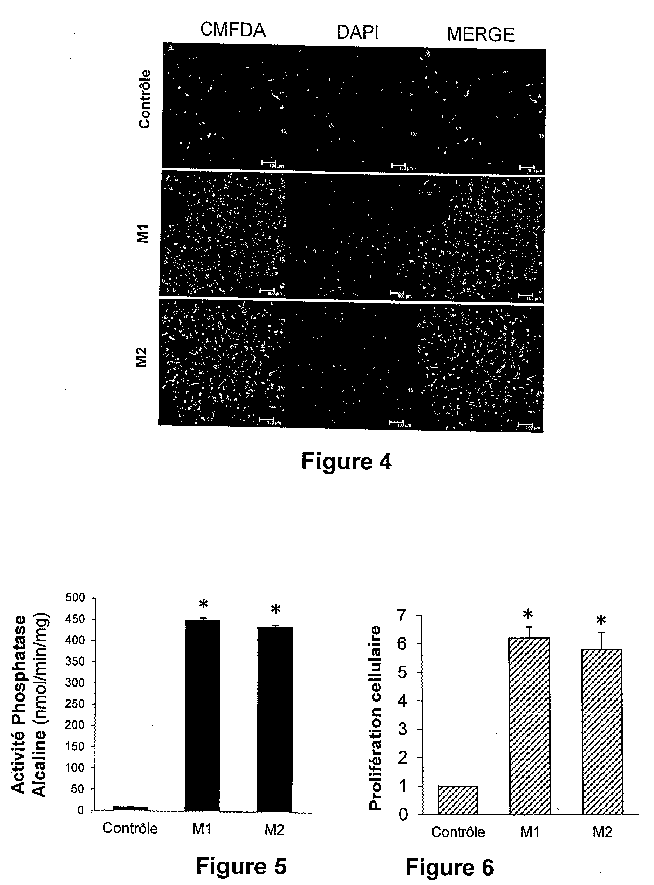

Après 48 heures de culture cellulaire sur les matériaux M1 et M2, les précurseurs ostéoblastiques ont été mis en évidence par marquage cytoplasmique au CMFDA. Les images obtenues en microscopie confocale sont présentées à la Fig. 4.

L'activité phosphatase alcaline a aussi été déterminée. Elle est multipliée par environ 400 dans les cellules pré-ostéoblastiques au contact des matériaux M1 et M2, par rapport à l'activité des cellules cultivées sur support céramique non modifié (Fig. 5) révélant une expression intense de la phosphatase alcaline. Une différence significative est observée entre les cellules cultivées sur le support céramique non modifié et les matériaux greffés M1 et M2. (**P*< 0.001).

La prolifération des précurseurs ostéoblastiques a été observée. Après 48 heures de culture cellulaire, le dédoublement de populations cellulaires est six fois supérieur sur les supports M1 et M2 comme le montre la Fig. 6 (**P*< 0.005).

Ces résultats démontrent un pouvoir régénérateur osseux intense développé par les matériaux M1 et M2, greffés par les peptides mimétiques de la protéine BMP-2, P1 et P2.

### 3 - Activité vis-à-vis des cellules endothéliales

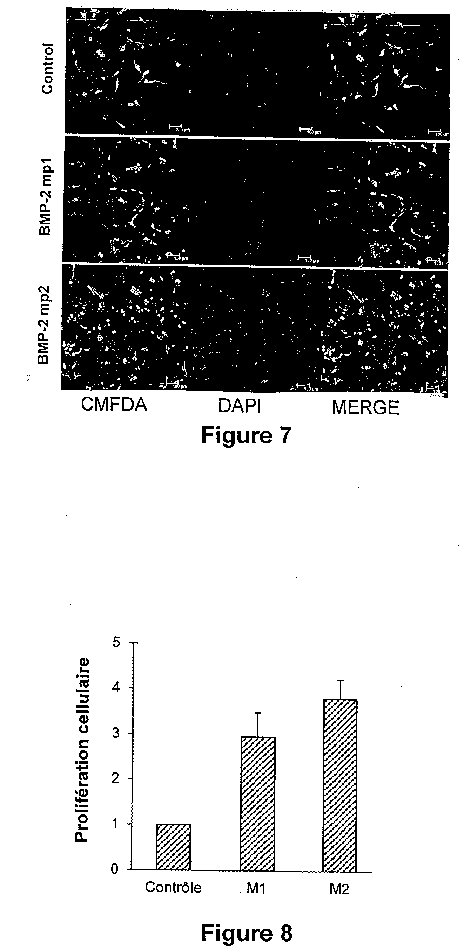

Des cellules endothéliales (type cellulaire présent dans la moelle osseuse au niveau des vaisseaux) ont été mises au contact des matériaux modifiés M1 et M2, et du support céramique non modifié pris comme référence. Après 48 heures de culture des cellules endothéliales (HUVEC, ATCC), une prolifération des cellules est observée (Fig. 7), qui est 3 fois supérieure sur les supports M1 et M2 que sur le support céramique non modifié (**P*< 0.005) (Fig. 8).

### 4 - Activité vis-à-vis des cellules de la moelle osseuse

Enfin, a été évalué l'effet des matériaux M1 et M2 au contact simultané des trois types de cellules ci-dessus, afin de prédire leur comportement une fois implantés *in vivo.*

Les tricultures cellulaires comprenant des cellules souches mésenchymateuse humaines de la moelle osseuse, des précurseurs ostéoblastiques et des cellules endothéliales, ont été mises en contact des matériaux M1 et M2 et d'un support non greffé pris pour référence, pendant 62 heures. Les images obtenues en microscopie confocale (non reproduites) montrent la formation de clusters cellulaires (ou réseaux de cellules) très importants.

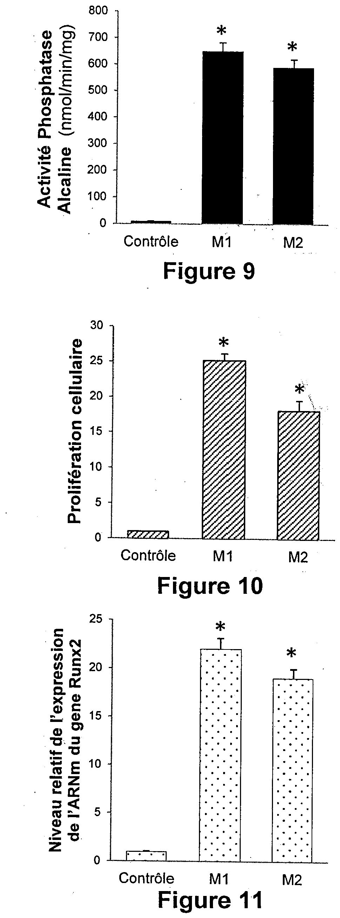

L'activité de la phosphatase alcaline a été déterminée après 62 heures de culture (Fig. 9). Une différence significative est observée entre les cellules cultivées sur des supports céramiques non modifiés et les matériaux greffés M1 et M2, puisqu'elle est 650 fois plus élevée (**P* < 0.01). L'expression du facteur de transcription ostéogénique Runx2 a été déterminée par la mesure de l'intensité de sa fluorescence dans la tri-culture cellulaire après 62 heures (**P*<0.05). Celle-ci est intense : vingt fois supérieure à la référence (Fig. 11). La prolifération cellulaire est accrue, avec un dédoublement des populations cellulaires vingt fois supérieur au contact des matériaux M1 et M2 qu'au contact du support non greffé (**P* < 0.005). Ceci démontre un pouvoir régénérateur intense des matériaux M1 et M2 (Fig. 10).

L'ensemble de ces résultats démontre la rapidité et l'intensité de l'activité ostéo-inductrice des supports céramiques greffées par les peptides mimétiques de la protéine BMP-2 selon l'invention.

### EXEMPLE 5 : Matériau nanoparticulaire greffé par le peptide mimétique P1

Dans le mode de réalisation présenté ci-après, le matériau obtenu est un gel de nanoparticules d'hydroxyapatite en suspension dans l'eau.

### Support :

Le support solide est constitué de nanoparticules d'hydroxyapatite (nano-bâtonnets de taille sub-micronique) se présentant à l'état de poudre, après lyophilisation. Ce sont des particules denses (non poreuses) caractérisées par une surface spécifique élevée.

### Peptide :

Le peptide P1 tel que décrit précédemment est utilisé.

### Mode opératoire :

Le protocole de greffage est identique à celui de l'Exemple 1. A l'issue des étapes a) et b), on obtient le matériau nanoparticulaire greffé NP1, à l'état de poudre.

Puis, la poudre greffée est mise en suspension dans l'eau, pour obtenir un gel de nanoparticules d'hydroxyapatite en suspension dans l'eau (5 mg/ml). Il est composé de nano-bâtonnets dans l'eau, avec un taux de matière sèche de l'ordre de 30%.

### Caractérisation : présence de la séquence peptidique

La composition atomique des nanoparticules greffées NP1 a été comparée à celle de nanoparticules non traitée (support solide pris comme référence). Les mesures ont été réalisées par spectroscopie à photoélectrons aux rayons X (Tableau 2).

**Tableau 2**

| **atome** | **Référence (% atomique)** | **Matériau NP1 (% atomique)** |
|---|---|---|
| P2p | 11,4 | 11,25 |
| C1s | 19,9 | 20,15 |
| Ca2p | 17,39 | 18,83 |
| O1s | 48,5 | 48,89 |
| N1s | non détecté | **3,61** |

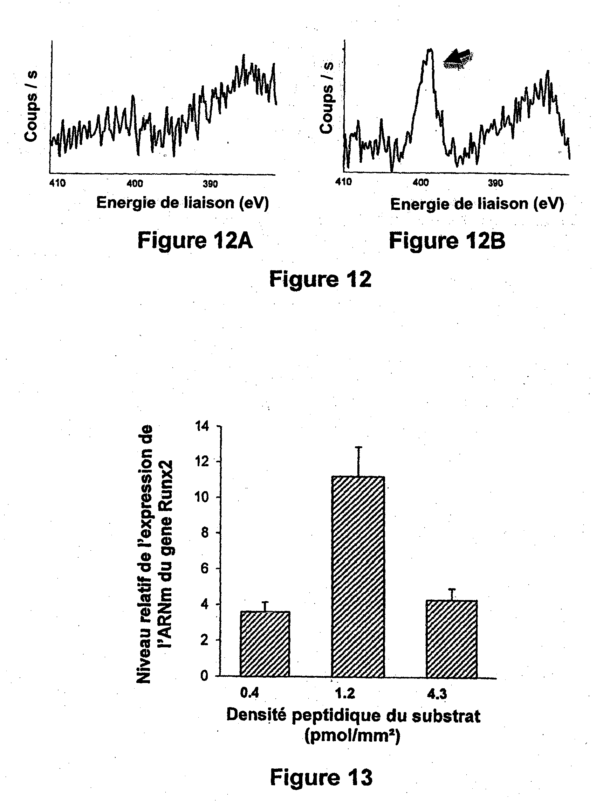

Le spectre d'azote (N1s) en surface des deux matériaux (NP1 et référence) obtenu par spectroscopie à photoélectrons aux rayons X est présenté aux Fig. 12A et 12B.

Les résultats montrent l'apparition dans le matériau NP1, d'un spectre d'azote à environ 4%, prouvant la présence de séquence peptidique sur le support nanoparticulaire.

### Caractérisation : densité en peptide greffé

La présence de peptides greffés en surface du matériau NP1 a été déterminée par microscopie à fluorescence à l'aide de peptides P1 fluorescents. Les résultats démontrent qu'un greffage covalent sur le support NP1 est bien obtenu. En outre, les images de microscopie électronique à transmission obtenues des nanoparticules céramiques aux différents stades du protocole de greffage montrent que la méthode de greffage n'a pas affecté les dimensions des nanoparticules. De manière plus générale c'est la possibilité d'un greffage peptidique sur des supports nanoparticulaires qui est validée.

### EXEMPLE 6 : Optimisation de la densité de greffage sur support modèle

Il a été démontré sur un support modèle synthétique, que l'on peut contrôler la densité de greffage sur les sites du support, et que la densité optimale peut être ainsi prédéterminée.

Le peptide P1 a été greffé de manière covalente sur un support modèle, à trois densités différentes. Le support est en polyéthylène téréphtalate (PET). Les densités sont de 0,4 pmol/mm², 1,2 pmol/mm² et 4,3 pmol/mm². Le taux d'expression du facteur Runx2 par RT-PCR en fonction de la densité du peptide P1 greffé en surface d'un support modèle a été mesuré pour ces trois densités (Fig. 13). Le meilleur niveau d'expression du gène Runx2 est obtenu pour 1,2 pmol/mm² de densité peptidique, ce qui correspond à une activité maximale de différenciation cellulaire pour le support modèle.

L'activité obtenue est en outre stable dans le temps (après 1 semaine de culture) : à 96h de culture, on observe une augmentation continue de l'expression du facteur Runx2 (environ 42%).

### EXEMPLE 7 : Optimisation de la densité de greffage sur support phosphocalcique

Les peptides P1 et P2 ont été greffés de manière covalente sur un support phosphocalcique, à trois densités différentes. Le support solide est un cube de céramique (65% d'hydroxyapatite et 35% de TCP), identique à celui de l'Exemple 1. Des densités greffage de 1,1 pmol/mm², 3,2 pmol/mm² et 4,9 pmol/mm² ont été obtenues. On démontre ainsi que l'on peut moduler et contrôler à volonté le taux de greffage des peptides d'intérêt sur les supports phosphocalciques.

Le taux d'expression du facteur Runx2 mesuré par RT-PCR, la prolifération cellulaire et l'activité de la phosphatase alcaline en fonction de la densité des peptides greffés en surface de ce support ont été mesurés pour les densités 3,2 et 4,9 pmol/mm². Les résultats d'expression du gène Runx2, de prolifération cellulaire et de l'activité de la phosphatase alcaline ont montré une activité de différentiation cellulaire marquée au regard des faibles densités de peptide greffé.

### SEQUENCE LISTING

**SEQ ID NO 1**
   LENGTH: 20
   TYPE: PRT
   ORGANISM: Artificial Sequence
   FEATURE:
      OTHER INFORMATION: Synthetic Peptide, Synthetized by FMOC/tBu solide-state peptide synthesis
   SEQUENCE: 1
      Lys IIe Pro Lys Ala Cys Cys Val Pro Thr Glu Leu Ser Ala IIe Ser Met Leu Tyr Leu
**SEQ ID NO 2**
   LENGTH: 21
   TYPE: PRT
   ORGANISM: Artificial Sequence
   FEATURE:
      OTHER INFORMATION: Synthetic Peptide, Synthetized by FMOC/tBu solide-state peptide synthesis
   SEQUENCE: 2
      Cys Lys Xaa₁ Pro Lys Xaa₂ Ser Ser Xaa₃ Pro
      Thr Glu Xaa₄ Ser Ala IIe Ser Met Leu Tyr Leu
**SEQ ID NO 3**
   LENGTH: 20
   TYPE: PRT
   ORGANISM: Artificial Sequence
   FEATURE:
      OTHER INFORMATION: Synthetic Peptide, Synthetized by FMOC/tBu solide-state peptide synthesis
   SEQUENCE: 3
      Lys Xaa₁ Pro Lys Xaa₂ Zaa₁ Zaa₂ Xaa₃ Pro
      Thr Glu Xaa₄ Ser Ala IIe Ser Met Leu Tyr Leu

## Revendications

1. Matériau de substitution osseuse pour la chirurgie osseuse et dentaire, *caractérisé en ce qu*'il comprend :
i) un support solide à base d'au moins un composé phosphocalcique présentant des groupements hydroxyles libres en surface, et
ii) une quantité d'un peptide mimétique de la protéine humaine BMP-2 de séquence
KIPKACCVPTELSAISMLYL
(SEQ ID No 1), ou d'un dérivé de ce peptide qui est un ligand interagissant avec le récepteur cellulaire BMP de type II, ledit dérivé ayant une séquence
KX₁PKX₂Z₁Z₂X₃PTEX₄SAISMLYL
(SEQ ID No 3) dans laquelle X₁, X₂, X₃ et X₄ sont des acides aminés non polaires, identiques ou différents, et Z₁ et Z₂ identiques ou différents, désignent un résidu cystéine ou sérine, ladite quantité de peptide mimétique de la protéine BMP-2 étant greffée de manière covalente par son extrémité N-terminale au niveau desdits groupements hydroxyles, avec une densité comprise entre 0,2.10⁻¹² mol/mm² et 100.10⁻¹² mol/mm² de surface du support solide.

2. Matériau selon la revendication 1, ***caractérisé en ce que*** la densité dudit peptide greffé à la surface du support solide est inférieure à 10.10⁻¹² mol/mm².

3. Matériau selon la revendication 2, ***caractérisé en ce que*** la densité dudit peptide greffé à la surface du support solide est comprise dans l'intervalle allant de 4,5.10⁻¹² mol/mm² à 7,5.10⁻¹² mol/mm².

4. Matériau selon l'une des revendications précédentes, ***caractérisé en ce que*** dans ledit peptide, Z₁ et Z₂ sont deux résidus sérine.

5. Matériau selon l'une des revendications précédentes, ***caractérisé en ce que*** le peptide est lié au support solide par l'intermédiaire d'une cystéine fixée à son extrémité N-terminale et d'un agent de couplage bifonctionnel comprenant un agent de fonctionnalisation de surface associé à un agent de liaison.

6. Matériau de substitution osseuse selon l'une des revendications précédentes, ***caractérisé en ce que*** ledit peptide est le peptide de séquence SEQ ID No 3, dans lequel :
X₁ = Ile, X₂ = Ala, X₃ = Val et X₄ = Leu.

7. Matériau de substitution osseuse selon l'une des revendications 1 à 5, ***caractérisé en ce que*** ledit peptide est le peptide de séquence SEQ ID No 3, dans lequel :
X₁ = Leu, X₂ = Val, X₃ = Leu et X₄ = Ala.

8. Matériau selon l'une des revendications précédentes, ***caractérisé en ce que*** ledit au moins un composé phosphocalcique est de l'hydroxyapatite, du phosphate tricalcique, ou un mélange de ceux-ci.

9. Matériau selon l'une des revendications précédentes, ***caractérisé en ce que*** le support solide est choisi parmi une céramique sous forme de granulés ou de blocs, une poudre amorphe, ou des nanoparticules phosphocalciques.

10. Procédé de préparation d'un matériau de substitution osseuse selon l'une des revendications précédentes, comprenant i) support solide à base d'au moins un composé phosphocalcique présentant des groupements hydroxyles libres en surface, et ii) une quantité d'un peptide mimétique de la protéine humaine BMP-2 de séquence
KIPKACCVPTELSAISMLYL
(SEQ ID No 1), ou d'un dérivé de ce peptide qui est un ligand du récepteur cellulaire de type II, ledit dérivé ayant une séquence
KX₁PKX₂Z₁Z₂X₃PTEX₄SAISMLYL
(SEQ ID No 3) dans laquelle X₁, X₂, X₃ et X₄ sont des acides aminés non polaires, identiques ou différents et Z₁ et Z₂ identiques ou différents, désignent un résidu cystéine ou sérine,
le procédé étant ***caractérisé en ce* qu'**il comprend les étapes consistant à :
a) faire réagir une partie des groupements hydroxyles dudit composé phosphocalcique avec un agent de fonctionnalisation puis avec un agent de liaison pour obtenir un support solide ayant une densité prédéterminée de sites modifiés en surface,
b) faire réagir le support solide ainsi modifié avec une solution contenant ledit peptide en excès pour obtenir un matériau ayant une densité prédéterminée comprise entre 0,2.10⁻¹² mol/mm² et 100.10⁻¹² mol/mm² de sites greffés par ledit peptide.

11. Procédé de préparation d'un matériau de substitution osseuse selon la revendication précédente ***caractérisé en ce que*** à l'étape a) on fait réagir une partie des groupements hydroxyles dudit composé phosphocalcique avec un agent de fonctionnalisation puis avec un agent de liaison pour obtenir, à l'étape b) un support solide ayant une densité prédéterminée de sites greffés en surface inférieure à 10.10⁻¹² mol/mm².

12. Procédé de préparation d'un matériau de substitution osseuse selon la revendication précédente ***caractérisé en ce que*** à l'étape a) on fait réagir une partie des groupements hydroxyles dudit composé phosphocalcique avec un agent de fonctionnalisation puis avec un agent de liaison pour obtenir, à l'étape b) un support solide ayant une densité prédéterminée de sites greffés en surface allant de 4,5.10⁻¹² mol/mm² à 7,5.10⁻¹² mol/mm².

13. Procédé de préparation d'un matériau de substitution osseuse selon l'une des revendications 10 à 12, ***caractérisé en ce que*** avant chacune des deux réactions de l'étape a), le support solide est séché à une température de l'ordre de 70°C à 100°C.

14. Procédé de préparation d'un matériau de substitution osseuse selon l'une des revendications 10 à 13, ***caractérisé en ce que**,* après l'étape b), on réalise un rinçage à l'eau du matériau de substitution osseuse obtenu jusqu'à élimination complète des peptides en excès.

15. Procédé de préparation d'un matériau de substitution osseuse selon l'une des revendications 10 à 14, ***caractérisé en ce que*** ledit support solide est choisi parmi :
- une céramique formulée en granulés ou en blocs, pour obtenir ledit matériau de substitution osseuse sous la même forme,
- une poudre amorphe apte à être mise en suspension dans une phase aqueuse pour obtenir ledit matériau de substitution osseuse sous forme d'un ciment de comblement,
- des nanoparticules aptes à être mises en suspension dans une phase aqueuse pour obtenir ledit matériau de substitution osseuse sous forme d'un gel de comblement.

## Patentansprüche

1. Knochenersatzmaterial für chirurgische Eingriffe an Knochen und Zähnen, ***dadurch gekennzeichnet, dass*** es Folgendes umfasst:
i) einen feststofflichen Träger auf Basis mindestens einer Calciumphosphatverbindung, an dessen Oberfläche sich freie Hydroxylgruppen befinden, und
ii) eine Menge eines Peptids, welches das menschliche Protein BMP-2 nachempfindet und die Sequenz
KIPKACCVPTELSAISMLYL
(SEQ-ID-Nr. 1) aufweist, oder eines Derivats dieses Peptids, wobei es sich um einen Liganden handelt, der mit dem BMP-Zellrezeptor des Typs II in Wechselwirkung tritt, wobei das Derivat eine Sequenz
KX₁PKX₂Z₁Z₂X₃PTEX₄SAISMLYL
(SEQ-ID-Nr. 3) aufweist, in welcher X₁, X₂, X₃ und X₄ für unpolare Aminosäuren gleicher oder unterschiedlicher Beschaffenheit stehen und Z₁ und Z₂, die von gleicher oder unterschiedlicher Beschaffenheit sind, einen Cystein- oder Serinrest bezeichnen, wobei die Menge an Peptid, welches das Protein BMP-2 nachempfindet, über ihr N-terminales Ende kovalent auf die Hydroxylgruppen gepfropft ist, mit einer Dichte im Bereich von 0,2.10⁻¹² mol/mm² bis 100.10⁻¹² mol/mm² der Oberfläche des feststofflichen Trägers.

2. Material nach Anspruch 1, ***dadurch gekennzeichnet, dass*** die Dichte des gepfropften Peptids an der Oberfläche des feststofflichen Trägers weniger als 10.10⁻¹² mol/mm² beträgt.

3. Material nach Anspruch 2, ***dadurch gekennzeichnet, dass*** die Dichte des gepfropften Peptids an der Oberfläche des feststofflichen Trägers im Bereich von 4,5.10⁻¹² mol/mm² bis 7,5.10⁻¹² mol/mm² liegt.

4. Material nach einem der vorhergehenden Ansprüche, ***dadurch gekennzeichnet, dass*** Z₁ und Z₂ in dem Peptid zwei Serinresten entsprechen.

5. Material nach einem der vorhergehenden Ansprüche, ***dadurch gekennzeichnet, dass*** die Bindung des Peptids an den feststofflichen Träger über ein Cystein erfolgt, welches an seinem N-terminalen Ende befestigt ist, und über ein Kupplungsmittel mit zwei funktionellen Gruppen, welches ein Mittel zur Einführung oberflächenständiger funktioneller Gruppen in Verbindung mit einem Bindemittel umfasst.

6. Knochenersatzmaterial nach einem der vorhergehenden Ansprüche, ***dadurch gekennzeichnet, dass*** es sich bei dem Peptid um das Peptid mit der Sequenz SEQ ID Nr. 3 handelt, wobei Folgendes gilt:
X₁ = Ile, X₂ = Ala, X₃ = Val und X₄ = Leu.

7. Knochenersatzmaterial nach einem der Ansprüche 1 bis 5, ***dadurch gekennzeichnet, dass*** es sich bei dem Peptid um das Peptid mit der Sequenz SEQ ID Nr. 3 handelt, wobei Folgendes gilt:
X₁ = Leu, X₂ = Val, X₃ = Leu und X₄ = Ala.

8. Material nach einem der vorhergehenden Ansprüche, ***dadurch gekennzeichnet, dass*** es sich bei der mindestens einen Calciumphosphatverbindung um Hydroxylapatit, Tricalciumphosphat oder eine Mischung derselben handelt.

9. Material nach einem der vorhergehenden Ansprüche, ***dadurch gekennzeichnet, dass*** der feststoffliche Träger aus einem Keramikwerkstoff in Form von Granulatkörnern oder Blöcken, einem amorphen Pulver oder Calciumphosphat-Nanopartikeln ausgewählt ist.

10. Verfahren zur Herstellung eines Knochenersatzmaterials, das einem der vorhergehenden Ansprüche entspricht und das i) einen feststofflichen Träger auf Basis mindestens einer Calciumphosphatverbindung umfasst, an dessen Oberfläche sich freie Hydroxylgruppen befinden, und ii) eine Menge eines Peptids, welches das menschliche Protein BMP-2 nachempfindet und welches die Sequenz
KIPKACCVPTELSAISMLYL
(SEQ-ID-Nr. 1) aufweist, oder eines Derivats dieses Peptids, wobei es sich um einen Liganden des BMP-Zellrezeptors des Typs II handelt, wobei das Derivat eine Sequenz
KX₁PKX₂Z₁Z₂X₃PTEX₄SAISMLYL
(SEQ-ID-Nr. 3) aufweist, in welcher X₁, X₂, X₃ und X₄ für unpolare Aminosäuren gleicher oder unterschiedlicher Beschaffenheit stehen und Z₁ und Z₂, die von gleicher oder unterschiedlicher Beschaffenheit sind, einen Cystein- oder Serinrest bezeichnen, wobei das Verfahren ***dadurch gekennzeichnet* ist, *dass*** es die folgenden Schritte umfasst:
a) Umsetzen eines Teils der Hydroxylgruppen der Calciumphosphatverbindung mit einem Mittel zur Einführung funktioneller Gruppen und danach mit einem Bindemittel, um einen feststofflichen Träger zu erhalten, an dessen Oberfläche sich modifizierte Stellen in einer vorbestimmten Dichte befinden,
b) Umsetzen des derart modifizierten feststofflichen Trägers mit einer Lösung, die das Peptid im Überschuss enthält, um ein Material mit einer vorbestimmten Dichte, welche im Bereich von 0,2.10⁻¹² mol/mm² bis 100.10⁻¹² mol/mm² liegt, an Stellen zu erhalten, die eine Pfropfung durch das Peptid aufweisen.

11. Verfahren zur Herstellung eines Knochenersatzmaterials nach dem vorhergehenden Anspruch, ***dadurch gekennzeichnet, dass*** in Schritt a) ein Teil der Hydroxylgruppen der Calciumphosphatverbindung mit einem Mittel zur Einführung funktioneller Gruppen und danach mit einem Bindemittel umgesetzt wird, um in Schritt b) einen feststofflichen Träger zu erhalten, an dessen Oberfläche sich gepfropfte Stellen in einer vorbestimmten Dichte von weniger als 10.10⁻¹² mol/mm² befinden.

12. Verfahren zur Herstellung eines Knochenersatzmaterials nach dem vorhergehenden Anspruch, ***dadurch gekennzeichnet, dass*** in Schritt a) ein Teil der Hydroxylgruppen der Calciumphosphatverbindung mit einem Mittel zur Einführung funktioneller Gruppen und danach mit einem Bindemittel umgesetzt wird, um in Schritt b) einen feststofflichen Träger zu erhalten, an dessen Oberfläche sich gepfropfte Stellen in einer vorbestimmten Dichte im Bereich von 4,5.10⁻¹² mol/mm² bis 7,5.10⁻¹² mol/mm² befinden.

13. Verfahren zur Herstellung eines Knochenersatzmaterials nach einem der Ansprüche 10 bis *12, **dadurch gekennzeichnet, dass*** der feststoffliche Träger vor jeder der beiden Umsetzungen des Schrittes a) bei einer Temperatur in der Größenordnung von 70 °C bis 100 °C getrocknet wird.

14. Verfahren zur Herstellung eines Knochenersatzmaterials nach einem der Ansprüche 10 bis 13, ***dadurch gekennzeichnet, dass*** das erhaltene Knochenersatzmaterial nach dem Schritt b) solange mit Wasser gespült wird, bis die überschüssigen Peptide vollständig entfernt sind.

15. Verfahren zur Herstellung eines Knochenersatzmaterials nach einem der Ansprüche 10 bis *14, **dadurch gekennzeichnet, dass*** der feststoffliche Träger aus den folgenden ausgewählt ist:
- einem Keramikwerkstoff, der als Granulat oder in Blöcken formuliert ist, um das Knochenersatzmaterial in ebendieser Form zu erhalten,
- einem amorphen Pulver, das dazu geeignet ist, in einer wässrigen Phase aufgeschlämmt zu werden, um das Knochenersatzmaterial in Form eines Füllzements zu erhalten,
- Nanopartikeln, die dazu geeignet sind, in einer wässrigen Phase aufgeschlämmt zu werden, um das Knochenersatzmaterial in Form eines Füllgels zu erhalten,

## Claims

1. Bone substitute material for bone and dental surgery, **characterized in that** it comprises:
i) a solid support made from at least one calcium phosphate compound having free hydroxyl groups at the surface, and
ii) an amount of a mimetic peptide of the human BMP-2 protein having the sequence
KIPKACCVPTELSAISMLYL
(SEQ ID No. 1), or of a derivative of this peptide which is a ligand that interacts with the type II BMP cell receptor, said derivative having a sequence
KX₁PKX₂Z₁Z₂X₃PTEX₄SAISMLYL
(SEQ ID No. 3) in which X₁, X₂, X₃ and X₄ are identical or different nonpolar amino acids and Z₁ and Z₂, which may be identical or different, denote a cysteine or serine residue, said amount of mimetic peptide of the BMP-2 protein being covalently grafted, via its N-terminal end, to said hydroxyl groups, with a density of from 0.2×10⁻¹² mol/mm² to 100×10⁻¹² mol/mm² of surface area of the solid support.

2. Material according to Claim 1, **characterized in that** the density of said peptide grafted at the surface of the solid support is less than 10×10⁻¹² mol/mm².

3. Material according to Claim 2, **characterized in that** the density of said peptide grafted at the surface of the solid support is included in the range of from 4.5×10⁻¹² mol/mm² to 7.5×10⁻¹² mol/mm².

4. Material according to one of the preceding claims, **characterized in that**, in said peptide, Z₁ and Z₂ are two serine residues.

5. Material according to one of the preceding claims, **characterized in that** the peptide is bonded to the solid support by means of a cysteine attached at its N-terminal end and of a bifunctional coupling agent having a surface-functionalizing agent combined with a linking agent.

6. Bone substitute material according to one of the preceding claims, **characterized in that** said peptide is the peptide having the sequence SEQ ID No. 3, in which:
X₁ = lie, X₂ = Ala, X₃ = Val and X₄ = Leu.

7. Bone substitute material according to one of Claims 1 to 5, **characterized in that** said peptide is the peptide having the sequence SEQ ID No. 3, in which:
X₁ = Leu, X₂ = Val, X₃ = Leu and X₄ = Ala.

8. Material according to one of the preceding claims, **characterized in that** said at least one calcium phosphate compound is hydroxyapatite, tricalcium phosphate, or a mixture thereof.

9. Material according to one of the preceding claims, **characterized in that** the solid support is chosen from amongst a ceramic in the form of granules or of blocks, an amorphous powder, or calcium phosphate nanoparticles.

10. Process for preparing a bone substitute material according to one of the preceding claims, comprising i) a solid support made from at least one calcium phosphate compound having free hydroxyl groups at the surface, and ii) an amount of a mimetic peptide of the human BMP-2 protein having the sequence
KIPKACCVPTELSAISMLYL
(SEQ ID No. 1), or of a derivative of this peptide which is a ligand of the type II cell receptor, said derivative having a sequence
K₁PKX₂Z₁Z₂X₃PTEX₄SAISMLYL
(SEQ ID No. 3) in which X₁, X₂, X₃ and X₄ are identical or different nonpolar amino acids and Z₁ and Z₂, which may be identical or different, denote a cysteine or serine residue, the process being **characterized in that** it comprises the steps consisting in:
a) reacting a part of the hydroxyl groups of said calcium phosphate compound with a functionalizing agent and then with a linking agent so as to obtain a solid support having a predetermined density of modified sites at the surface,
b) reacting the solid support thus modified with a solution containing said peptide in excess so as to obtain a material having a predetermined density of from 0.2×10⁻¹² mol/mm² to 100×10⁻¹² mol/mm² of sites grafted with said peptide.

11. Process for preparing a bone substitute material according to the preceding claim, **characterized in that**, in step a), some of the hydroxyl groups of said calcium phosphate compound are reacted with a functionalizing agent and then with a linking agent so as to obtain, in step b), a solid support having a predetermined density of sites grafted at the surface of less than 10×10⁻¹² mol/mm².

12. Process for preparing a bone substitute material according to the preceding claim, **characterized in that**, in step a), some of the hydroxyl groups of said calcium phosphate compound are reacted with a functionalizing agent and then with a linking agent so as to obtain, in step b), a solid support having a predetermined density of sites grafted at the surface ranging from 4.5×10⁻¹² mol/mm² to 7.5×10⁻¹² mol/mm².

13. Process for preparing a bone substitute material according to one of Claims 10 to 12, **characterized in that**, before each of the two reactions of step a), the solid support is dried at a temperature of about 70°C to 100°C.

14. Process for preparing a bone substitute material according to one of Claims 10 to 13, **characterized in that**, after step b), rinsing with water of the bone substitute material obtained is carried out until there is complete elimination of the excess peptides.

15. Process for preparing a bone substitute material according to one of Claims 10 to 14, **characterized in that** said solid support is chosen from amongst:
- a ceramic formulated in granules or in blocks, so as to obtain said bone substitute material in the same form,
- an amorphous powder capable of being suspended in an aqueous phase so as to obtain said bone substitute material in the form of a filling cement,
- nanoparticles capable of being suspended in an aqueous phase so as to obtain said bone substitute material in the form of a filling gel.


### SEQUENCE LISTING

<110> TEKNIMED SA
<120> SUBSTITUTS OSSEUX GREFFES PAR des PEPTIDES MIMETIQUES DE LA PROTEINE HUMAINE BMP-2
<130> F1 T36 31 B1 F5
<140> FR1352302
   <141> 2013-03-14
<160> 3
<170> PatentIn version 3.5
<210> 1
   <211> 20
   <212> PRT
   <213> Artificial Sequence
<220>
   <223> Synthetic Construct
<400> 1
   Lys Ile Pro Lys Ala Cys Cys Val Pro Thr Glu Leu Ser Ala Ile Ser
   1               5                   10                  15
   Met Leu Tyr Leu
               20
<210> 2
   <211> 21
   <212> PRT
   <213> Artificial Sequence
<220>
   <223> Synthetic Construct
<220>
   <221> misc_feature
   <222> (3)..(3)
   <223> Xaa can be any naturally occurring amino acid
<220>
   <221> misc_feature
   <222> (6) .. (6)
   <223> Xaa can be any naturally occurring amino acid
<220>
   <221> misc_feature
   <222> (9) .. (9)
   <223> Xaa can be any naturally occurring amino acid
<220>
   <221> misc_feature
   <222> (13)..(13)
   <223> Xaa can be any naturally occurring amino acid
<400> 2
   Cys Lys Xaa Pro Lys Xaa Ser Ser Xaa Pro Thr Glu Xaa Ser Ala Ile
   1               5                   10                  15
   Ser Met Leu Tyr Leu
               20
<210> 3
   <211> 20
   <212> PRT
   <213> Artificial Sequence
<220>
   <223> Synthetic Construct
<220>
   <221> misc_feature
   <222> (2) .. (2)
   <223> Xaa can be any naturally occurring amino acid
<220>
   <221> misc_feature
   <222> (5)..(5)
   <223> Xaa can be any naturally occurring amino acid
<220>
   <221> misc_feature
   <222> (6) .. (7)
   <223> Xaa can be a Serine or a Cysteine
<220>
   <221> misc_feature
   <222> (8)..(8)
   <223> Xaa can be any naturally occurring amino acid
<220>
   <221> misc_feature
   <222> (12)..(12)
   <223> Xaa can be any naturally occurring amino acid
<400> 3
   Lys Xaa Pro Lys Xaa Xaa Xaa Xaa Pro Thr Glu Xaa Ser Ala Ile Ser
   1               5                   10                  15
   Met Leu Tyr Leu
               20